# EUROPÄISCHE PATENTSCHRIFT

(11) **EP 0 826 361 B1**
(45) Veröffentlichungstag und Bekanntmachung des Hinweises auf die Patenterteilung: **13.11.2002**
(21) Anmeldenummer: 97113930.8
(22) Anmeldetag: 13.08.1997
(51) Int. Cl.: A61K 7/42

(54) **Photostabile UV-A-Filter enthaltende kosmetische Zubereitungen**
Cosmetical preparations containing photostable UV-A filters
Préparations cosmétiques contenant des filtres UV-A photostables

(30) Priorität: 23.08.1996 DE 19634229
(43) Veröffentlichungstag der Anmeldung: 04.03.1998
(73) Patentinhaber: BASF AKTIENGESELLSCHAFT, 67056 Ludwigshafen (DE)
(72) Erfinder: Habeck, Thorsten, Dr., 67149 Meckenheim (DE); Aumüller, Alexander, Dr., 67435 Neustadt (DE); Schehlmann, Volker, Dr., 67354 Römerberg (DE); Westenfelder, Horst, 67435 Neustadt (DE); Wünsch, Thomas, Dr., 67346 Speyer (DE)

(56) Entgegenhaltungen:
- DE-A- 2 118 798
- FR-A- 1 251 590
- US-A- 3 280 069

## Beschreibung

Die Erfindung betrifft die Verwendung von α-Acyl-, β-Arylacrylnitrilen als photostabile UV-A-Filter in kosmetischen Zubereitungen zum Schutz der menschlichen Epidermis gegen UV-Strahlung, speziell im Bereich von 320 bis 400 nm.

Die in kosmetischen Zubereitungen eingesetzten Lichtschutzmittel haben die Aufgabe, schädigende Einflüsse des Sonnenlichts auf die menschliche Haut zu verhindern oder zumindest in ihren Auswirkungen zu reduzieren. Daneben dienen diese Lichtschutzmittel aber auch dem Schutz weiterer Inhaltsstoffe vor Zerstörung oder Abbau durch UV-Strahlung.

Das an die Erdoberfläche gelangende Sonnenlicht hat einen Anteil an UV-B- (280 bis 320 nm) und an UV-A-Strahlung (> 320 nm), welche sich direkt an den Bereich des sichtbaren Lichtes anschließen. Der Einfluß auf die menschliche Haut macht sich besonders bei der UV-B-Strahlung durch Sonnenbrand bemerkbar. Dementsprechend bietet die Industrie eine größere Zahl von Substanzen an, welche die UV-B-Strahlung absorbieren und damit den Sonnenbrand verhindern.

Nun haben dermatologische Untersuchungen gezeigt, daß auch die UV-A-Strahlung durchaus Hautschädigungen hervorrufen kann, indem beispielsweise das Keratin oder Elastin geschädigt wird. Hierdurch werden Elastizität und Wasserspeichervermögen der Haut reduziert, d.h. die Haut wird weniger geschmeidig und neigt zur Faltenbildung. Die auffallend hohe Hautkrebshäufigkeit in Gegenden starker Sonneneinstrahlung zeigt, daß offenbar auch Schädigungen der Erbinformationen in den Zellen durch Sonnenlicht, speziell durch UV-A-Strahlung, hervorgerufen werden. All diese Erkenntnisse lassen daher die Entwicklung effizienter Filtersubstanzen für den UV-A-Bereich notwendig erscheinen.

Es besteht ein wachsender Bedarf an Lichtschutzmitteln für kosmetische Zubereitungen, die vor allem als UV-A-Filter dienen können und deren Absorptionsmaxima deshalb im Bereich von ca. 320 bis 380 nm liegen sollten. Um mit einer möglichst geringen Einsatzmenge die gewünschte Wirkung zu erzielen, sollten derartige Lichtschuztmittel zusätzlich eine hoch spezifische Extinktion aufweisen. Außerdem müssen Lichtschutzmittel für kosmetische Präparate noch eine Vielzahl weiterer Anforderungen erfüllen, beispielsweise gute Löslichkeit in kosmetischen Ölen, hohe Stabilität der mit ihnen hergestellten Emulsionen, toxikologische Unbedenklichkeit sowie geringen Eigengeruch und geringe Eigenfärbung.

Eine weitere Anforderung, der Lichtschutzmittel genügen müssen, ist eine ausreichende Photostabilität. Dies ist aber mit den bisher verfügbaren UV-A absorbierenden Lichtschutzmitteln nicht oder nur unzureichend gewährleistet.

In der französischen Patentschrift Nr. 2 440 933 wird das 4-(1,1-Dimethylethyl)-4'-methoxydibenzoylmethan als UV-A-Filter beschrieben. Es wird vorgeschlagen, diesen speziellen UV-A-Filter, der von der Firma GIVAUDAN unter der Bezeichnung "PARA-SOL 1789" verkauft wird, mit verschiedenen UV-B-Filtern zu kombinieren, um die gesamten UV-Strahlen mit einer Wellenlänge von 280 bis 380 nm zu absorbieren.

Dieser UV-A-Filter ist jedoch, wenn er allein oder in Kombination mit UV-B-Filtern verwendet wird, photochemisch nicht beständig genug, um einen anhaltenden Schutz der Haut während eines längeren Sonnenbades zu gewährleisten, was wiederholte Anwendungen in regelmäßigen und kurzen Abständen erfordert, wenn man einen wirksamen Schutz der Haut gegen die gesamten UV-Strahlen erzielen möchte.

Deshalb sollen gemäß EP 0514491 die nicht ausreichend photostabilen UV-A-Filter durch den Zusatz von 2-Cyan-3,3-diphenylacrylsäureestern stabilisiert werden, die selbst im UV-B-Bereich als Filter dienen.

Weiterhin wurde gemäß EP 251 398 schon vorgeschlagen, UV-A- und UV-B-Strahlung absorbierende Chromophore durch ein Bindeglied in einem Molekül zu vereinen. Dies hat den Nachteil, daß einerseits keine freie Kombination von UV-A- und UV-B-Filtern in der kosmetischen Zubereitung mehr möglich ist und daß Schwierigkeiten bei der chemischen Verknüpfung der Chromophore nur bestimmte Kombinationen zulassen.

US 3,280,069 beschreibt die Verwendung von u.a. 3,5-Di-tert. butyl-4-hydroxy-zimt säureester zur Stabilisierung von Kunststoffen gegenüben Oxidation und zum Schutz gegen UV-Licht.

Es bestand daher die Aufgabe, Lichtschutzmittel für kosmetische Zwecke vorzuschlagen, die im UV-A-Bereich absorbieren und die photostabil sind.

Diese Aufgabe wurde erfindungsgemäß gelöst durch Verwendung von Verbindungen der Formel I 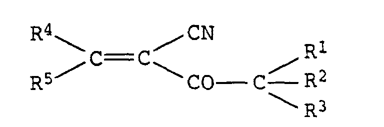 in der R¹, R² und R³ Wasserstoff, aliphatische oder cycloaliphatische Reste, insbesondere Alkyl-, Alkenyl-, Cycloalkyl- oder Cycloalkenyl-Reste mit jeweils bis zu 18 C-Atomen bedeuten, wobei jeweils zwei der Reste R¹, R² und R³ zusammen einen 5- oder 6-Ring bilden können, in der R⁵ für Wasserstoff steht, und R⁴ für gegebenenfalls substituiertes Thienyl oder für einen gegebenenfalls substituierten 4-Hydroxy- oder 4-Alkoxyphenylrest steht, als UV-A-Filter in kosmetischen Zubereitungen zum Schutz der menschlichen Haut gegen Sonnenstrahlen, allein oder zusammen mit an sich für kosmetische Zubereitungen bekannten, im UV-Bereich, insbesondere im UV-B-Bereich, absorbierenden Verbindungen.

Vorzugsweise bedeuten R¹, R² und R³ niedermolekulare Alkylreste, z.B. mit 1 bis 4 C-Atomen.

Als Alkoxyreste kommen solche mit 1 bis 12 C-Atomen, vorzugsweise mit 1 bis 8 C-Atomen in Betracht.

### Beispielsweise sind zu nennen:

| | |
|---|---|
| methoxy | isopropoxy- |
| n-propoxy- | 1-methylpropoxy- |
| n-butoxy- | n-pentoxy- |
| 2-methylpropoxy- | 3-methylbutoxy- |
| 1,1-dimethylpropoxy- | 2,2-dimethylpropoxy- |
| hexoxy- | 1-methyl-1-ethylpropoxy- |
| heptoxy- | octoxy- |
| 2-ethylhexoxy- | |

Die erfindungsgemäß zu verwendenden Verbindungen sind zum Teil aus der chemischen Literatur bekannt.

Neue Verbindungen sind solche der Formel II 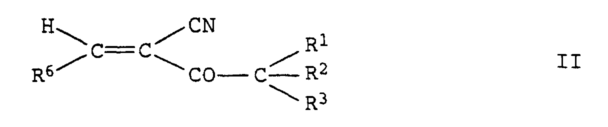 in der R¹, R² und R³ niedermolekulare Alkylreste, z.B. mit 1 bis 4 C-Atomen, und R⁶ einen gegebenenfalls durch niedermolekulare Alkylreste substituierten 2-Thienylrest oder einen 4-(3,5-di-t.-Butyl)-1-hydroxy- oder alkoxyphenylrest bedeuten.

Die erfindungsgemäß zu verwendenden Verbindungen der Formel I oder II können nach der Gleichung 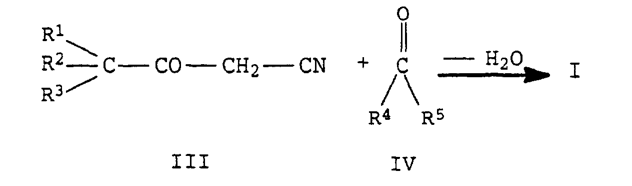 durch Kondensation hergestellt werden, wobei R¹ bis R⁵ die oben genannte Bedeutungen haben.

Die Lichtschutzmittel enthaltenden kosmetischen Zubereitungen sind in der Regel auf der Basis eines Trägers, der mindestens eine Ölphase enthält. Es sind aber auch Zubereitungen allein auf wässriger (Gel)Basis möglich. Demgemäß kommen Öle, Öl-in-Wasserund Wasser-in-Öl-Emulsionen, Cremes und Pasten, Lippenschutzstiftmassen oder Gele in Betracht.

Solche Sonnenschutzpräparate können demgemäß in flüssiger, pastöser oder fester Form vorliegen, beispielsweise als Wasser-in-Öl-Cremes, Öl-in-Wasser-Cremes und -Lotionen, Aerosol-Schaumcremes, Gele, Öle, Fettstifte, Puder, Sprays oder alkoholisch-wäßrige Lotionen.

Übliche Ölkomponenten in der Kosmetik sind beispielsweise Paraffinöl, Glycerylstearat, Isopropylmyristat, Diisopropyladipat, 22-Ethylhexansäurecetylstearylester, hydriertes Polyisobuten, Vaseline, Caprylsäure/Caprinsäure-Triglyceride, mikrokristallines Wachs, Lanolin und Stearinsäure.

Übliche kosmetische Hilfsstoffe, die als Zusätze in Betracht kommen können, sind z.B. Emulgatoren wie Fettalkoholethoxylate, Sorbitanfettsäureester oder Lanolinderivate, Verdickungsmittel wie Carboxymethylcellulose oder vernetzte Polyacrylsäure, Konservierungsmittel und Parfüms. Schließlich können weitere an sich bekannte im UV-A-Bereich absorbierenden Substanzen mitverwendet werden, sofern sie im Gesamtsystem der erfindungsgemäß zu verwendenden Kombination aus UV-B und UV-A Filter stabil sind.

Gegenstand der vorliegenden Erfindung sind weiterhin kosmetische Zubereitungen, die 0,1 bis 10 Gew.-%, vorzugsweise 1 bis 7 Gew.-%, bezogen auf die gesamte Menge der kosmetischen Zubereitung, eine oder mehrere der Verbindungen der Formel I zusammen mit an sich für kosmetische Zubereitungen bekannten, im UV-B-Bereich absorbierenden Verbindungen als Lichtschutzmittel enthalten, wobei die Verbindungen der Formel I in der Regel in geringerer Menge als die UV-B-absorbierenden Verbindungen eingesetzt werden.

Der größte Teil der Lichtschutzmittel in den zum Schutz der menschlichen Epidermis dienenden kosmetischen Zubereitungen besteht aus Verbindungen, die UV-Licht im UV-B-Bereich absorbieren d.i. im Bereich von 280 bis 320 nm. Beispielsweise beträgt der Anteil der erfindungsgemäß zu verwendenden UV-A-Absorber 10 bis 90 Gew.-%, bevorzugt 20 bis 50 Gew.-% bezogen auf die Gesamtmenge von UV-B und UV-A absorbierenden Substanzen.

Als UV-B-Filtersubstanzen, die in Kombination mit den erfindungsgemäß zu verwendenden Verbindungen der Formel I angewandt werden, kommen beliebige UV-B-Filtersubstanzen in Betracht. Beispielsweise sind zu nennen:

| Nr. | Stoff | CAS-Nr. (=Säure) |
|---|---|---|
| 1 | 4-Aminobenzoesäure | 150-13-0 |
| 2 | 3-(4'Trimethylammonium)-benzylidenbornan-2-on-methylsulfat | 52793-97-2 |
| 3 | 3,3,5-Trimethyl-cyclohexyl-salicylat (Homosalatum) | 118-56-9 |
| 4 | 2-Hydroxy-4-methoxy-benzophenon (Oxybenzonum) | 131-57-7 |
| 5 | 2-Phenylbenzimidazol-5-sulfonsäure und ihre Kalium-, Natrium- u. Triethanolaminsalze | 27503-81-7 |
| 6 | 3,3'-(1,4-Phenylendimethin)-bis(7,7-dimethyl-2-oxobicyclo[2.2.1]heptan-1-methansulfonsäure) und ihre Salze | 90457-82-2 |
| 7 | 4-Bis(polyethoxy)amino-benzoesäurepolyethoxyethylester | 113010-52-9 |
| 8 | 4-Dimethylamino-benzoesäure-2-ethylhexylester | 21245-02-3 |
| 9 | Salicylsäure-2-ethylhexylester | 118-60-5 |
| 10 | 4-Methoxy-zimtsäure-2-isoamylester | 7/6/7-10-2 |
| 11 | 4-Methoxy-zimtsäure-2-ethylhexylester | 5466-77-3 |
| 12 | 2-Hydroxy-4-methoxy-benzophenon-5-sulfon-(Sulisobenzonum) und das Natriumsalz | 4065-45-6 |
| 13 | 3-(4'-Sulfo)benzyliden-bornan-2-on und Salze | 58030-58-6 |
| 14 | 3-(4'-Methyl)benzyliden-bornan-2-on | 36861-47-9 |
| 15 | 3-Benzylidenbornan-2-on | 16087-24-8 |
| 16 | 1-(4'-Isopropylphenyl)-3-phenylpropan-1,3-dion | 63260-25-9 |
| 17 | 4-Isopropylbenzylsalicylat | 94134-93-7 |
| 18 | 2,4,6-Trianilin-(o-carbo-2'-ethylhexyl-1'-oxy)-1,3,5-triazin | 88122-99-0 |
| 19 | 3-Imidazol-4-yl-acrylsäure und ihr Ethylester | 104-98-3* |
| 20 | 2-Cyano-3,3-diphenylacrylsäureethylester | 5232-99-5 |
| 21 | 2-Cyano-3,3-diphenylacrylsäure-2'-ethylhexylester | 6197-30-4 |
| 22 | Menthyl-o-aminobenzoate oder: 5-Methyl-2-(1-methylethyl)-2-aminobenzoate | 134-09-8 |
| 23 | Glyceryl p-aminobenzoat oder: 4-Aminobenzoesäure-1-glyceryl-ester | 136-44-7 |
| 24 | 2,2'-Dihydroxy-4-methoxybenzophenon (Dioxybenzone) | 131-53-3 |
| 25 | 2-Hydroxy-4-methoxy-4-methylbenzophenon (Mexonon) | 1641-17-4 |
| 26 | Triethanolamin Salicylat | 2174-16-5 |
| 27 | Dimethoxyphenylglyoxalsäure oder: 3,4-dimethoxy-phenyl-glyoxal-saures Natrium | |
| 28 | 3-(4'Sulfo)benzyliden-bornan-2-on und seine Salze | 56039-58-8 |

Schließlich sind auch mikronisierte Pigmente wie Titandioxid und Zinkoxid zu nennen.

Die erfindungsgemäß zu verwendenden Verbindungen zeichnen sich in der Regel durch ein besonders hohes Absorptionsvermögen im Bereich der UV-A-Strahlung aus. Weiterhin sind sie gut in kosmetischen Ölen löslich und lassen sich leicht in kosmetische Formulierungen einarbeiten. Die mit den Verbindungen I hergestellten Emulsionen zeichnen sich besonders durch ihre hohe Stabilität, die Verbindungen I selber durch ihre hohe Photostabilität aus, und die mit I hergestellten Zubereitungen durch ihr angenehmes Hautgefühl aus.

### Beispiele:

### Beispiel 1 (Herstellung)

### Allgemeine Vorschrift:

Ein Mol der Verbindung der Formel III (Cyanmethylenketon) wird mit einem Mol der Verbindung der Formel IV (Aldehyd bzw. Keton) in Methanol gelöst, die Lösung mit je 10 ml Piperidin und Eisessig versetzt und zum Rückfluß erhitzt. Nach ca. 4 h ist die Reaktion beendet. Man saugt vom ausgefallenen Produkt ab, das aus Methanol umkristallisiert wird. Ausbeute ca. 60 - 70 %.

Die so hergestellten Verbindungen sind in Tabelle 1 angegeben.

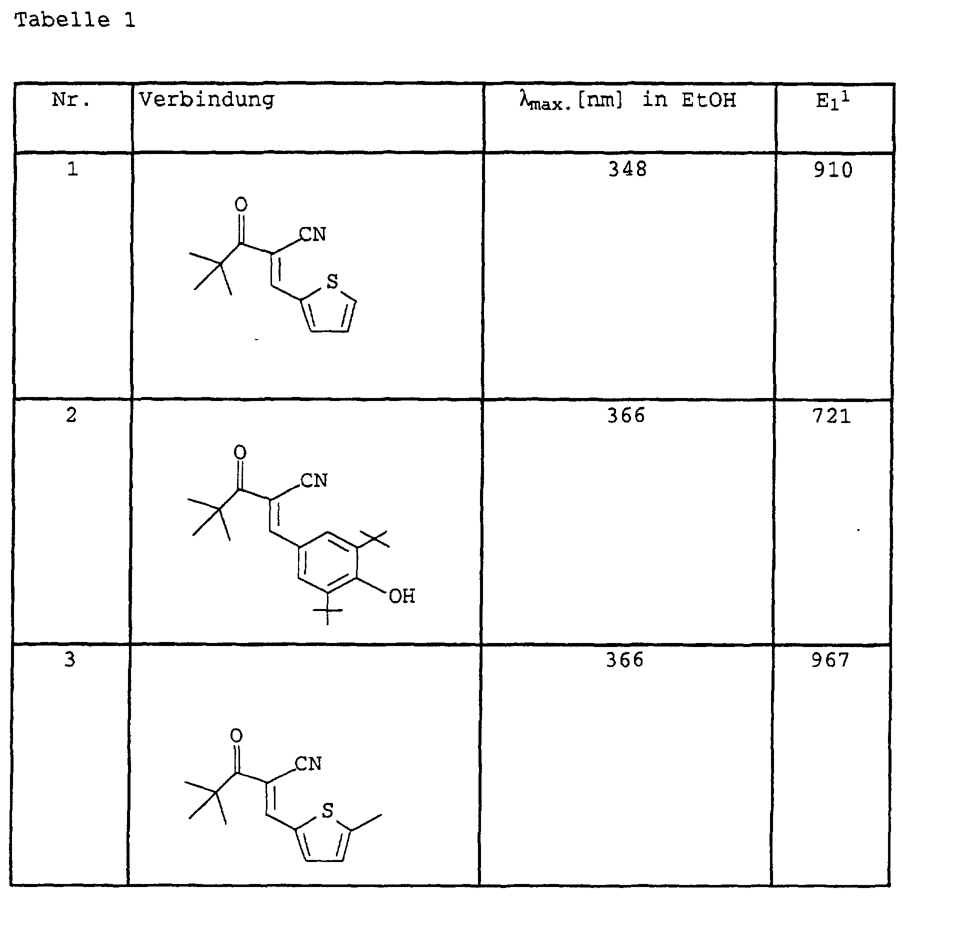

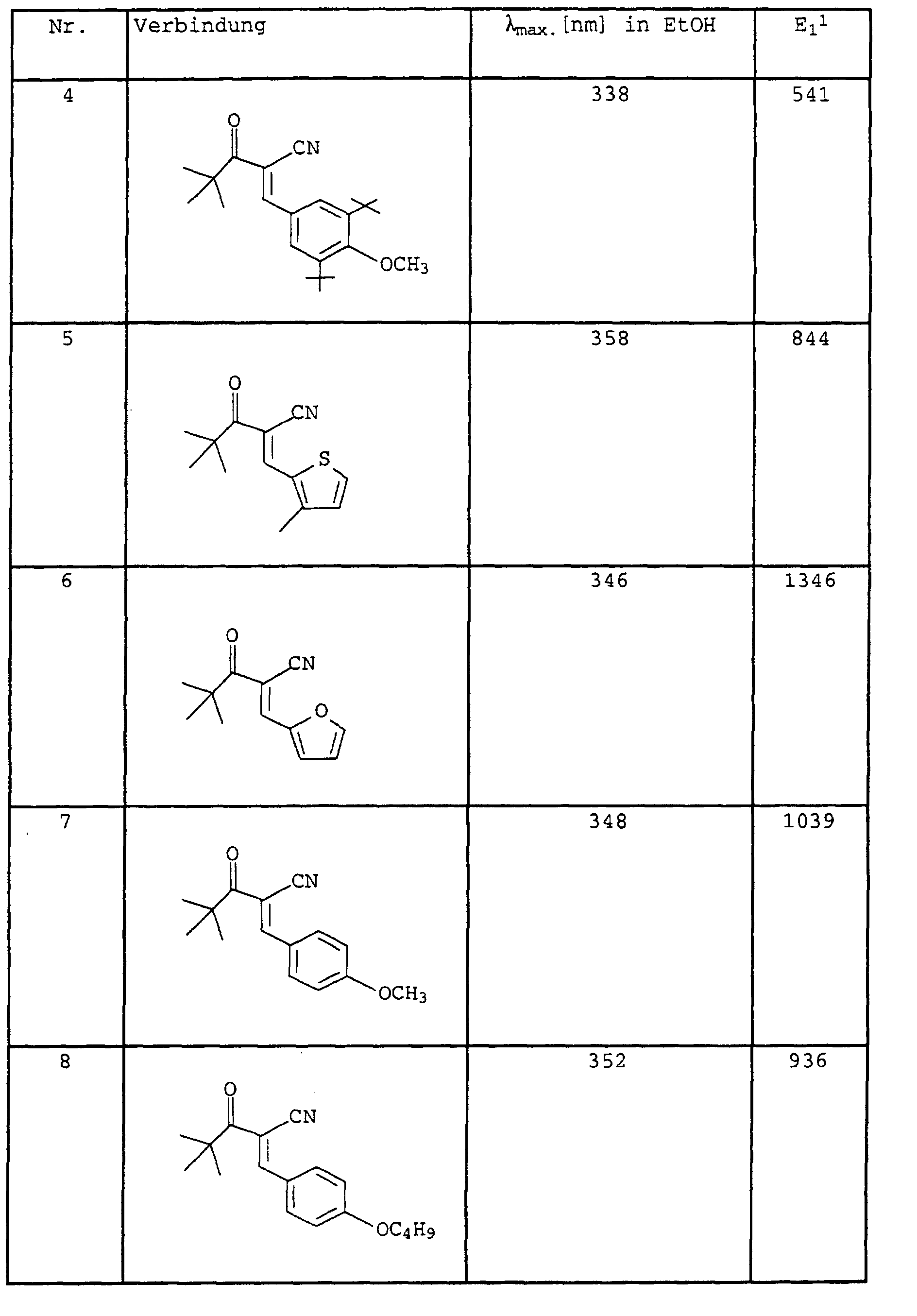

### Allgemeine Herstellvorschrift zur Herstellung von Emulsionen für kosmetische Zwecke

Alle öllöslichen Bestandteile werden in einem Rührkessel auf 85°C erwärmt. Wenn alle Bestandteile geschmolzen sind, bzw. als Flüssigphase vorliegen, wird die Wasserphase unter Homogenisierer eingearbeitet. Unter Rühren wird die Emulsion auf ca. 40°C abgekühlt, parfümiert, homogenisiert und dann unter ständigem Rühren auf 25°C abgekühlt.

### Zubereitungen

### Beispiel 2

Zusammensetzung für die Lippenpflege

| | |
|---|---|
| ad 100 | Eucerinum anhydricum |
| 10,00 | Glycerin |
| 10,00 | Titanium Dioxid |
| 0,5-10 | Verbindung der Tabelle 1, Nr. 1 |
| 8,00 | Octyl Methoxycinnamat |
| 5,00 | Zink Oxid |
| 4,00 | Castoröl |
| 4,00 | Pentaerythrithil Stearat/caprat/Caprylat Adipat |
| 3,00 | Glyceryl Stearat SE |
| 2,00 | Bienenwachs |
| 2,00 | Microkristallines Wachs |
| 2,00 | Quaternium-18 Bentonit |
| 1,50 | PEG-45/Dodecyl Glycol Copolymer |

### Beispiel 3

Zusammensetzung für Sunblocker mit Mikropigmenten

| | |
|---|---|
| ad 100 | Wasser |
| 10,00 | Octyl Methoxcinnamat |
| 6,00 | PEG-7-Hydrogenated Castor Öl |
| 6,00 | Titanium Dioxid |
| 0,5-10 | Verbindung der Tabelle 1, Nr. 2 |
| 5,00 | Mineral Öl |
| 5,00 | Isoamyl p-Methoxycinnamat |
| 5,00 | Propylen Glycol |
| 3,00 | Jojoba Öl |
| 3,00 | 4-Methylbenzyliden Campher |
| 2,00 | PEG-45/Dodecyl Glycol Copolymer |
| 1,00 | Butyl Methoxydibenzoylmethan |
| 1,00 | Dimethicon |
| 0,50 | PEG-40-Hydrogenated Castor Öl |
| 0,50 | Tocopheryl Acetat |
| 0,50 | Phenoxyethanol |
| 0,20 | EDTA |

### Beispiel 4

Fettfreies Gel

| | |
|---|---|
| ad 100 | Wasser |
| 8,00 | Octyl Methoxycinnamat |
| 7,00 | Titanium Dioxid |
| 0,5-10 | Verbindung der Tabelle 1, Nr. 3 |
| 5,00 | Glycerin |
| 5,00 | PEG-25 PABA |
| 1,00 | 4-Methylbenzyliden Campher |
| 0,40 | Acrylate C10-C30 Alkyl Acrylat Crosspolymer |
| 0,30 | Imidazolidinyl Urea |
| 0,25 | Hydroxyethyl Cellulose |
| 0,25 | Sodium Methylparaben |
| 0,20 | Disodium EDTA |
| 0,15 | Fragrance |
| 0,15 | Sodium Propylparaben |
| 0,10 | Sodium Hydroxid |

### Beispiel 5

Sonnencreme (LSF 20)

| | |
|---|---|
| ad 100 | Wasser |
| 8,00 | Octyl Methoxycinnamat |
| 8,00 | Titanium Dioxid |
| 6,00 | PEG-7-Hydrogenated Castor Öl |
| 0,5-10 | Verbindung der Tabelle 1, Nr. 1 |
| 6,00 | Mineral Öl |
| 5,00 | Zink Oxid |
| 5,00 | Isopropyl Palmitat |
| 5,00 | Imidazolidinyl Urea |
| 3,00 | Jojoba Öl |
| 2,00 | PEG-45/Dodecyl Glycol Copolymer |
| 1,00 | 4-Methylbenzyliden Campher |
| 0,60 | Magnesium Stearat |
| 0,50 | Tocopheryl Acetat |
| 0,25 | Methylparaben |
| 0,20 | Disodium EDTA |
| 0,15 | Propylparaben |

### Beispiel 6

Sonnencreme wasserfest

| | |
|---|---|
| ad 100 | Wasser |
| 8,00 | Octyl Methoxycinnamat |
| 5,00 | PEG-7-Hydrogenated Castor Öl |
| 5,00 | Propylene Glycol |
| 4,00 | Isopropyl Palmitat |
| 4,00 | Caprylic/Capric Triglycerid |
| 0,5-10 | Verbindung nach Tabelle 1, Nr. 1 |
| 4,00 | Glycerin |
| 3,00 | Jojoba Öl |
| 2,00 | 4-Methylbenzyliden Campher |
| 2,00 | Titanium Dioxid |
| 1,50 | PEG-45/Dodecyl Glycol Copolymer |
| 1,50 | Dimethicon |
| 0,70 | Magnesium Sulfat |
| 0,50 | Magnesium Stearat |
| 0,15 | Fragrance |

### Beispiel 7

Sonnenmilch (LSF 6)

| | |
|---|---|
| ad 100 | Wasser |
| 10,00 | Mineral Öl |
| 6,00 | PEG-7-Hydrogenated Castor Öl |
| 5,00 | Isopropyl Palmitat |
| 3,50 | Octyl Methoxycinnamat |
| 0,5-10 | Verbindung nach Tabelle 1, Nr. 2 |
| 3,00 | Caprylic/Capric Triglycerid |
| 3,00 | Jojoba Öl |
| 2,00 | PEG-45/Dodecyl Glycol Copolymer |
| 0,70 | Magnesium Sulfat |
| 0,60 | Magnesium Stearat |
| 0,50 | Tocopheryl Acetat |
| 0,30 | Glycerin |
| 0,25 | Methylparaben |
| 0,15 | Propylparaben |
| 0,05 | Tocopherol |

## Patentansprüche

1. Verwendung von Verbindungen der Formel I 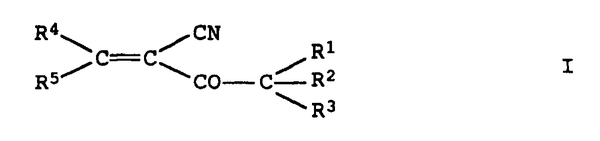 in der R¹, R² und R³ Wasserstoff oder aliphatische oder cycloaliphatische Reste mit jeweils bis zu 18 C-Atomen bedeuten, wobei jeweils zwei der Reste R¹, R² und R³ zusammen einen 5- oder 6-Ring bilden können, R⁴ einen substituierten oder unsubstituierten Thienyl-, 4-Hydroxy- oder 4-Alkoxyphenylrest bedeutet und R⁵ für Wasserstoff steht, als UV-A-Filter in kosmetischen Zubereitungen zum Schutz der menschlichen Haut gegen Sonnenstrahlen, allein oder zusammen mit an sich für kosmetische Zubereitungen bekannten, im UV-Bereich absorbierenden Verbindungen.

2. Verwendung von Verbindungen der Formel I gemäß Anspruch 1, wobei R⁵ für Wasserstoff steht und R¹, R² und R³ niedermolekulare Alkylreste mit 1 bis 4 C-Atomen bedeuten.

3. Verwendung von Verbindungen der Formel I gemäß Anspruch 1, wobei R⁵ für Wasserstoff steht, R¹, R² und R³ niedermolekulare Alkylreste mit 1 bis 4 C-Atomen bedeuten und R⁴ für substituiertes oder unsubstituiertes Thienyl steht.

4. Verwendung von Verbindungen der Formel I gemäß Anspruch 1, wobei R¹, R² und R³ niedermolekulare Alkylreste mit 1 bis 4 C-Atomen bedeuten, R⁵ für Wasserstoff und R⁴ für einen substituierten oder unsubstituierten 4-Hydroxy- oder 4-Alkoxyphenylrest steht.

5. Lichtschutzmittel enthaltende kosmetische Zubereitungen zum Schutz der menschlichen Epidermis gegen UV-Licht im Bereich von 280 bis 400 nm, **dadurch gekennzeichnet, daß** sie in einem kosmetisch geeigneten Träger, allein oder zusammen mit an sich für kosmetische Zubereitungen bekannten im UV-Bereich absorbierenden Verbindungen, als photostabile UV-A-Filter wirksame Mengen von Verbindungen der Formel I 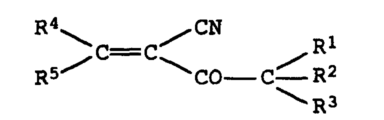 enthalten, in der R¹, R² und R³ Wasserstoff oder aliphatische oder cycloaliphatische Reste mit jeweils bis zu 18 C-Atomen bedeuten, wobei jeweils zwei der Reste R¹, R² und R³ zusammen eine 5- oder 6-Ring bilden können und R⁴ einen substituierten oder unsubstituierten Thienyl-, 4-Hydroxy- oder 4-Alkoxyphenylrest bedeutet und R⁵ für Wasserstoff steht.

6. Lichtschutzmittel gemäß Anspruch 5, enthaltend als UV-A-Filter Verbindungen der Formel I, wobei R⁵ für Wasserstoff steht und R¹, R² und R³ niedermolekulare Alkylreste mit 1 bis 4 C-Atomen bedeuten.

7. Lichtschutzmittel gemäß Anspruch 5, enthaltend als UV-A-Filter Verbindungen der Formel I, wobei R⁵ für Wasserstoff steht, R¹, R² und R³ niedermolekulare Alkylreste mit 1 bis 4 C-Atomen bedeuten und R⁴ für substituiertes oder unsubstituiertes Thienyl steht.

8. Lichtschutzmittel gemäß Anspruch 5, enthaltend als UV-A-Filter Verbindungen der Formel I, wobei R¹, R² und R³ niedermolekulare Alkylreste mit 1 bis 4 C-Atomen bedeuten, R⁵ für Wasserstoff und R⁴ für einen substituierten oder unsubstituierten 4-Hydroxy- oder 4-Alkoxyphenylrest steht.

9. Lichtschutzmittel enthaltende kosmetische Zubereitungen gemäß Anspruch 5, in denen der kosmetisch geeignete Träger mindestens eine Ölphase enthält.

10. Neue Verbindungen der Formel II, 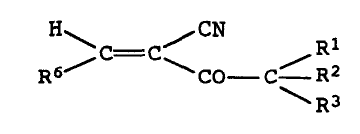 in der R¹, R² und R³ niedermolekulare Alkylreste mit 1 bis 4 C-Atomen und R⁶ einen gegebenenfalls durch niedermolekulare Alkylreste substituierten 2-Thienylrest oder einen 4-(3,5-dit.-Butyl)-alkoxyphenylrest bedeuten.

## Claims

1. The use of compounds of the formula I 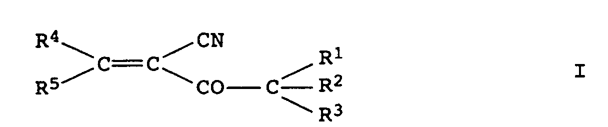 where R¹, R² and R³ are hydrogen or aliphatic or cycloaliphatic radicals each having up to 18 carbon atoms, it being possible for any two of the radicals R¹, R² and R³ together to form a 5- or 6-membered ring, R⁴ is a substituted or unsubstituted thienyl, 4-hydroxy- or 4-alkoxyphenyl radical, and R⁵ is hydrogen, as UV-A filters in cosmetic preparations for protecting the human skin from the sun's rays, alone or together with compounds which absorb in the UV region and are known for cosmetic preparations.

2. The use of compounds of the formula I as claimed in claim 1, where R⁵ is hydrogen and R¹, R² and R³ are low molecular weight alkyl radicals having from 1 to 4 carbon atoms.

3. The use of compounds of the formula I as claimed in claim 1, where R⁵ is hydrogen, R¹, R² and R³ are low molecular weight alkyl radicals having from 1 to 4 carbon atoms, and R⁴ is substituted or unsubstituted thienyl.

4. The use of compounds of the formula I as claimed in claim 1, where R¹, R² and R³ are low molecular weight alkyl radicals having from 1 to 4 carbon atoms, R⁵ is hydrogen and R⁴ is a substituted or unsubstituted 4-hydroxy- or 4-alkoxyphenyl radical.

5. A cosmetic preparation which comprises a sunscreen agent for protecting the human epidermis against UV light in the range from 280 to 400 nm, which comprises, in a cosmetically suitable carrier, alone or together with compounds which absorb in the UV region and are known for cosmetic preparations, as photostable UV-A filters effective amounts of compounds of the formula I 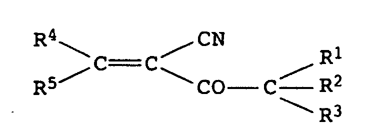 where R¹, R² and R³ are hydrogen or aliphatic or cycloaliphatic radicals each having up to 18 carbon atoms, it being possible for any two of the radicals R¹, R² and R³ together to form a 5- or 6-membered ring, and R⁴ is a substituted or unsubstituted thienyl, 4-hydroxy- or 4-alkoxyphenyl radical, and R⁵ is hydrogen.

6. A sunscreen agent as set forth in claim 5, comprising as UV-A filters compounds of the formula I where R⁵ is hydrogen and R¹, R² and R³ are low molecular weight alkyl radicals having from 1 to 4 carbon atoms.

7. A sunscreen agent as set forth in claim 5, comprising as UV-A filters compounds of the formula I where R⁵ is hydrogen, R¹, R² and R³ are low molecular weight alkyl radicals having from 1 to 4 carbon atoms and R⁴ is substituted or unsubstituted thienyl.

8. A sunscreen agent as set forth in claim 5, comprising as UV-A filters compounds of the formula I where R¹, R² and R³ are low molecular weight alkyl radicals having from 1 to 4 carbon atoms, R⁵ is hydrogen and R⁴ is a substituted or unsubstituted 4-hydroxy- or 4-alkoxyphenyl radical.

9. A cosmetic preparation which comprises a sunscreen agent as claimed in claim 5, in which the cosmetically suitable carrier contains at least one oil phase.

10. A novel compound of the formula II 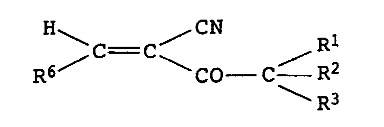 where R¹, R² and R³ are low molecular weight alkyl radicals having from 1 to 4 carbon atoms and R⁶ is a 2-thienyl radical which is unsubstituted or substituted by low molecular weight alkyl radicals, or is a (3,5-di-t-butyl)-4-alkoxyphenyl radical.

## Revendications

1. Utilisation de composés de formule I 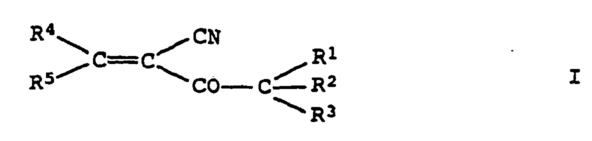 dans laquelle R¹, R² et R³ représentent l'hydrogène ou des restes aliphatiques ou cycloaliphatiques ayant chacun jusqu'à 18 atomes de C, tandis qu'à chaque fois deux des restes R¹, R² et R³ peuvent former ensemble un cycle à 5 ou 6 chaînons, R⁴ désigne un reste thiényle, 4-hydroxy- ou 4-alcoxyphényle, substitué ou non-substitué, et R⁵ désigne l'hydrogène, en tant que filtre UV-A dans des préparations cosmétiques pour la protection de la peau humaine contre les rayons solaires, seuls ou en mélange avec des composés absorbant dans le domaine UV, connus en soi pour des préparations cosmétiques.

2. Utilisation de composés de formule I selon la revendication 1, tandis que R⁵ désigne l'hydrogène et R¹, R² et R³ représentent des restes alkyle de faible masse moléculaire ayant 1 à 4 atomes de C.

3. Utilisation de composés de formule I selon la revendication 1, tandis que R⁵ désigne l'hydrogène, R¹, R² et R³ représentent des restes alkyle de faible masse moléculaire ayant 1 à 4 atomes de C, et R⁴ désigne un thiényle substitué ou non-substitué.

4. Utilisation de composés de formule I selon la revendication 1, tandis que R¹, R² et R³ représentent des restes alkyle de faible masse moléculaire ayant 1 à 4 atomes de C, R⁵ représente l'hydrogène et R⁴ désigne un reste 4-hydroxy- ou 4-alcoxyphényle substitué ou non-substitué.

5. Produits de protection contre la lumière contenant des préparations cosmétiques pour la protection de l'épiderme humain contre la lumière UV dans l'intervalle de 280 à 400 nm, **caractérisé par le fait qu'**ils contiennent dans un support approprié du point de vue cosmétique, seuls ou conjointement avec des composés connus en soi pour des préparations cosmétiques, absorbants dans le domaine des UV, des quantités actives comme filtre UV-A photostable, de composés de formule I 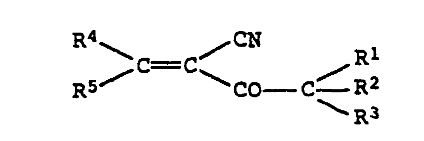 dans laquelle R¹, R² et R³ représentent l'hydrogène ou des restes aliphatiques ou cycloaliphatiques ayant chacun jusqu'à 18 atomes de C, tandis qu'à chaque fois deux des restes R¹, R² et R³ peuvent former ensemble un cycle à 5 ou 6 chaînons et R⁴ désigne- un reste thiényle, 4-hydroxy- ou 4-alcoxyphényle, substitué ou non-substitué, et R⁵ désigne l'hydrogène.

6. Produits de protection contre la lumière selon la revendication 5, contenant comme filtre UV-A des composés de formule I, où R⁵ désigne l'hydrogène et R¹, R² et R³ représentent des restes alkyle de faible masse moléculaire ayant 1 à 4 atomes de C.

7. Produits de protection contre la lumière selon la revendication 5, contenant comme filtre UV-A des composés de formule I, où R⁵ désigne l'hydrogène, R¹, R² et R³ représentent des restes alkyle de faible masse moléculaire ayant 1 à 4 atomes de C, et R⁴ désigne un thiényle substitué ou non-substitué.

8. Produits de protection contre la lumière selon la revendication 5, contenant comme filtre UV-A des composés de formule I, où R¹, R² et R³ représentent des restes alkyle de faible masse moléculaire ayant 1 à 4 atomes de C, R⁵ représente l'hydrogène et R⁴ désigne un reste 4-hydroxy- ou 4-alcoxyphényle substitué ou non-substitué.

9. Préparations cosmétiques contenant des produits de protection contre la lumière selon la revendication 5, dans lesquelles le support approprié du point de vue cosmétique contient au moins une phase huileuse.

10. Nouveaux composés de formule II, 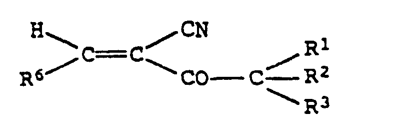 dans laquelle R¹, R² et R³ désignent des restes alkyle de faible masse moléculaire ayant 1 à 4 atomes de C et R⁶ représente un reste 2-thiényle ou un reste 4-(3,5-di-tert-butyl)-alcoxyphényle, éventuellement substitué par des restes alkyle de faible masse moléculaire.
